# EUROPEAN PATENT APPLICATION

(11) **EP 4 610 354 A1**
(43) Date of publication of application: **03.09.2025**
(21) Application number: 23877073.9
(22) Date of filing: 14.09.2023
(51) Int. Cl.: C12N 15/09

(54) **GENOME EDITING METHOD AND COMPOSITION FOR GENOME EDITING**

(30) Priority: 14.10.2022 JP 2022165722
(71) Applicant: Inplanta Innovations Inc., Yokohama-shi, Kanagawa 230-0052 (JP); National Institute of Advanced Industrial Science and Technology, Chiyoda-ku Tokyo 100-8921 (JP); TOPPAN Holdings Inc., Taito-ku Tokyo (JP)
(72) Inventor: TERAKAWA, Teruhiko, Yokohama-shi, Kanagawa 230-0052 (JP); YANO, Tsubasa, Yokohama-shi, Kanagawa 230-0052 (JP); MITSUDA, Nobutaka, Tsukuba-shi, Ibaraki 305-8560 (JP); NAKAMURA, Akiyoshi, Tsukuba-shi, Ibaraki 305-8560 (JP); SUGANO, Shigeo, Tsukuba-shi, Ibaraki 305-8560 (JP); ITO, Seiichiro, Tokyo 112-8531 (JP); MAKINO, Yoichi, Tokyo 112-8531 (JP)
(74) Representative: Jones, Nicholas Andrew
(86) International application number: PCT/JP2023/033481
(87) International publication number: WO 2024/080067

(57) **Abstract**

Provided is novel genome editing means that allows recognition of new PAM sequences in eukaryotic cell genomes, to modify the genomes in a site-specific manner. The method is a method for modifying a target DNA sequence in a genome of eukaryotic cells in a site-specific manner, wherein the method comprises introducing into the eukaryotic cells:
(1) Cas protein recognizing a PAM (protospacer adjacent motif) sequence containing the nucleotide sequence 5'-NNACNN-3' listed as SEQ ID NO: 1 (where each "N" represents any one nucleotide independently selected from among adenine, cytosine, thymine and guanine), or nucleic acid coding for the Cas protein, and
(2) guide RNA that can hybridize to the target DNA sequence in the genome while also forming a complex with the Cas protein, and that can direct sequence-specific binding of the complex to the target DNA sequence, or nucleic acid coding for the guide RNA,
to modify the eukaryotic cell genome at the target DNA sequence.

## Description

### FIELD

The present invention relates to the field of genetic engineering, and specifically to the field of genome editing. More specifically, the invention relates to a novel genome editing method for modifying a target DNA sequence in a eukaryotic cell genome in a site-specific manner, and genome editing means, such as a composition for genome editing, to be used in the method.

### BACKGROUND

Genome editing techniques using site-specific DNA-modifying proteins are known. In recent years, techniques for applying CRISPR/Cas (Clustered Regularly Interspaced Short Palindromic Repeats/CRISPR associated protein) systems for genome editing have been developed and implemented. CRISPR/Cas systems are of type I, type II and type III, with type II being most commonly used for genome editing. In a type II CRISPR/Cas system, the nuclease Cas9 is used as an RNA-guided nuclease (RGN). Cas9 has come to be used for manipulation of the genomes of a variety of eukaryotic cells (such as those of fish, plants and humans) (NPL 1). Cas9 is generally used as a complex with guide RNA. By introducing Cas9/guide RNA complexes into cells it is possible to cause mutations in target genes of a variety of animals and plants. By forming a complex with two RNAs, known as Cas9 CRISPR RNA (crRNA) and trans-activating crRNA (tracrRNA), it functions as an active endonuclease, cleaving exogenous genetic factors in phages or plasmids into which it has infiltrated. A CRISPR/Cas system is therefore useful for simply synthesizing short sgRNA having a sequence homologous to the target DNA sequence, but is also advantageous, particularly in a type II CRISPR/Cas system, for allowing genome editing using the simple protein Cas9.

Cas9 recognizes PAM (protospacer adjacent motif) sequences in DNA, cleaving double-stranded DNA in a blunt end manner upstream from them. The length and nucleotide sequence of the PAM sequence recognized by Cas9 varies depending on the type of bacteria from which Cas9 derives. For example, Cas9 from *Streptococcus pyogenes* (SpyCas9) recognizes the three nucleotides 5'-NGG-3' as a PAM sequence, allowing cleavage of the sequence upstream from the two guanine residues (PTL 1) ("N" in a nucleotide sequence mentioned herein represents any nucleotide, unless otherwise specified). *Streptococcus thermophilus* has two different forms of Cas9, which recognize respective sequences of 5 to 6 nucleotides: 5'-NGGNG-3' or 5'-NNAGAA-3' as PAM sequences (PTL 2).

### [CITATION LIST]

### [PATENT LITERATURE]

[PTL 1] International Patent Publication No. WO2014/093661
[PTL 2] Japanese Patent Public Inspection No. 2015-510778

### [NON PATENT LITERATURE]

[NPL 1] Charpentier and Doudna, Nature, 2013,495:50-51

### SUMMARY

### [TECHNICAL PROBLEM]

While the CRISPR/Cas system is an excellent means for genome editing, greater diversity of recognition of PAM sequences is desired in order to increase its utility.

Having been devised in light of the problems described above, the present invention has as its object to provide novel genome editing means that allows recognition of new PAM sequences in eukaryotic cell genomes, for site-specific modification of said genomes.

### [SOLUTION TO PROBLEM]

As a result of much diligent research directed toward solving the aforementioned problem, the present inventors found a Cas protein that recognizes a novel PAM sequence containing 5'-NNACNN-3', and found that by expressing the Cas protein together with guide RNA in eukaryotic cells having a target DNA sequence in their genome it is possible to modify the eukaryotic cell genome in a site-specific manner based on the novel target DNA sequence, and the invention has been completed upon this finding.

Specifically, the present invention encompasses the following.

### [Aspect 1]

A method for modifying a target DNA sequence in a genome of eukaryotic cells in a site-specific manner, wherein the method comprises introducing into the eukaryotic cells:
(1) Cas protein recognizing a PAM (protospacer adjacent motif) sequence containing the nucleotide sequence 5'-NNACNN-3' listed as SEQ ID NO: 1 (where each "N" represents any one nucleotide independently selected from among adenine, cytosine, thymine and guanine), or nucleic acid coding for the Cas protein, and
(2) guide RNA that can hybridize to the target DNA sequence in the genome while also forming a complex with the Cas protein, and that can direct sequence-specific binding of the complex to the target DNA sequence, or nucleic acid coding for the guide RNA, to modify the eukaryotic cell genome at the target DNA sequence.

### [Aspect 2]

The method according to Aspect 1, wherein the Cas protein is derived from an *Abyssicoccus* bacterium, and preferably *Abyssicoccus albus.*

### [Aspect 3]

The method according to Aspect 1 or Aspect 2, wherein the Cas protein includes a nuclear localization signal (NLS).

### [Aspect 4]

The method according to any one of Aspect 1 to Aspect 3, wherein the Cas protein includes an amino acid sequence with at least 80%, at least 85%, at least 90%, at least 92%, at least 95%, at least 96%, at least 97%, at least 98% or at least 99% sequence similarity to the amino acid sequence listed as SEQ ID NO: 4.

### [Aspect 5]

The method according to any one of Aspect 1 to Aspect 4, wherein the Cas protein has endonuclease activity.

### [Aspect 6]

The method according to any one of Aspect 1 to Aspect 5, wherein the PAM sequence includes the nucleotide sequence 5'-NNACGN-3' listed as SEQ ID NO: 2 or the nucleotide sequence 5'-NNACAN-3' listed as SEQ ID NO: 3.

### [Aspect 7]

The method according to any one of Aspect 1 to Aspect 6, wherein the guide RNA is single-stranded guide RNA (sgRNA), and also includes crRNA and tracrRNA.

### [Aspect 8]

The method according to Aspect 7, wherein the crRNA includes a spacer sequence with a nucleotide length of 15 to 30, which can form a double strand with a complementary strand of the target DNA.

### [Aspect 9]

The method according to Aspect 7 or Aspect 8, wherein the crRNA includes a stem-loop with a nucleotide length of 12 to 36.

### [Aspect 10]

The method according to any one of Aspect 1 to Aspect 9, wherein the eukaryotic cells are animal cells, plant cells or microbial cells.

### [Aspect 11]

The method according to any one of Aspect 1 to Aspect 10, wherein the nucleotide sequence of the nucleic acid coding for the Cas protein is codon-optimized for expression in animal cells, plant cells or microbial cells.

### [Aspect 12]

A composition for genome editing to be used in the method according to any one of Aspect 1 to Aspect 11, the composition comprising the Cas protein or nucleic acid coding for the Cas protein.

### [Aspect 13]

The composition according to Aspect 12, which further comprises guide RNA or nucleic acid coding for the guide RNA.

### [ADVANTAGEOUS EFFECTS OF INVENTION]

According to the genome editing method of the invention it is possible to modify a eukaryotic cell genome in a site-specific manner based on a novel target DNA sequence, by using Cas protein recognizing a novel PAM sequence that includes 5'-NNACNN-3'.

### BRIEF DESCRIPTION OF DRAWINGS

Fig. 1 is a drawing schematically depicting the construction of a vector for production of AalCas9 protein in Example 1.
Fig. 2 is a drawing showing the construction of the nucleotide sequence of sgRNA (SEQ ID NO: 23) for Example 2 and Example 3.
Fig. 3 is a graph showing analysis results for the PAM domain in Example 2.
Fig. 4 is a photograph showing an example of electrophoresis results for the reaction product of an *in vitro* DNA cleavage assay in Example 3. The left side of the photograph shows the results of incubating the reaction mixture for 60 minutes at 25°C, and the right side shows the results of incubating the reaction mixture for 60 minutes at 37°C.
Fig. 5A and 5B are graphs showing time-dependent change in AalCas9 activity in an *in vitro* DNA cleavage assay for Example 3. The graph of Fig. 5A shows the results of incubating the reaction mixture at 25°C, and the graph of Fig. 5B shows the results of incubating the reaction mixture at 37°C.
Fig. 6A and 6B shows results of genomic DNA editing in *Arabidopsis thaliana* protoplast cells by AalCas9 in Example 4. Fig. 6A shows the editing rate for each target DNA, and Fig. 6B shows mutant DNA determined using a next-generation sequencer.
Fig. 7 shows genomic DNA editing rates in *Arabidopsis thaliana* plant bodies by AalCas9 in Example 5.
Fig. 8 shows genome editing rates in human cultured cells HEK293FT by AalCas9 in Example 6.

### DESCRIPTION OF EMBODIMENTS

The invention will now be described in greater detail by concrete embodiments. However, the invention is not restricted to the described embodiments and may be carried out in any form within a range that is not outside of the gist of the invention.

All of the references including patent literature (such as patent applications and patent publications) and non-patent literature cited for the purpose of the invention are invoked in their entirety and are incorporated herein for all purposes.

Unless otherwise specified, the terms used herein throughout are understood to have the same meanings as used in the relevant field.

Information relating to the sequences of publicly known proteins or genes, among the proteins or genes described below, can be obtained from a well-known database such as GenBank of the NCBI (National Center for Biotechnology Information).

Unless specifically mentioned otherwise herein, "A" in the nucleotide sequences represents adenine, "G" represents guanine, "C" represents cytosine and "T" represents thymine, and "N" represents an arbitrary nucleotide selected from the group consisting of adenine, cytosine, thymine and guanine.

### · Genome editing method:

One aspect of the invention relates to a genome editing method for site-specific modification of a target DNA sequence in a eukaryotic cell genome (hereunder also referred to as "genome editing method of the invention", or "method of the invention", as appropriate). The main feature of this method is that it comprises introducing into eukaryotic cells:
(1) Cas protein recognizing a specific PAM sequence, or nucleic acid coding for the Cas protein, and
(2) guide RNA, or nucleic acid coding for the guide RNA,
to modify the eukaryotic cell genome at the target DNA sequence.

### · Cells:

The cells referred to herein are not particularly restricted and may be either prokaryotic cells or eukaryotic cells, but are preferably eukaryotic cells. Examples of prokaryotic cells include bacteria and archaebacteria. Examples of eukaryotic cells include microbial cells, plant cells and animal cells. Microbial cells include cells of various microorganisms such as yeast, fungi and protozoans. Plant cells include cells from various plants including spermatophytes, ferns and mosses and algae. Animal cells include cells derived from various animals such as vertebrates (mammals, birds, reptiles, amphibians and fish), arthropods (such as insects), and mollusks. Specific examples of mammalian cells include CHO cells, HeLa cells, HEK293 cells and COS-1 cells. The specific manner of cells is not particularly restricted and may be cells in the body (*in vivo*), cultured cells (*in vitro*), primary cultured cells (*in vitro* and *ex vivo*), and grafted cells. Such cells are commonly used in the technical field.

### · Genome editing:

The term "genome editing" as used herein means a technique for editing a genome by using site-specific DNA-modifying protein to introduce a desired modification into target sites of the genomes of different types of cells. Examples of the "modification" referred to here include, but are not limited to, cleaving a specific gene in the genome to disrupt the gene, inserting or substituting a DNA fragment into a target site of the genome, and introducing point mutations in a highly efficient manner to modify the gene function. The term "target site" here means a specified site in the eukaryotic cell genome that is to be edited. The term "cleave" refers to cleavage of the covalently bonded main chain of a nucleotide molecule.

The target site for genome editing may be selected as appropriate depending on the type of site-specific DNA modifying protein described below. For example, when using Cas9 as a type of Cas protein, as a site-specific DNA modifying protein, the target site for genome editing is preferably a site in genomic DNA comprising a DNA strand (target strand) consisting of a PAM sequence and a sequence of predetermined base length (such as about 20 nucleotides) adjacent to its 5'-end, and a complementary DNA strand (non-target strand).

One example of a target site on a genome to be edited, though not limitative, is all or a portion of a gene in a eukaryotic cell genome which is a gene to be modified or disrupted, or a region overlapping with or adjacent to the gene.

Specific examples of genes to be edited include genes related to primary metabolism (such as amino acids), genes related to secondary metabolism (such as flavonoids and polyphenols), glucose metabolism related genes, lipid metabolism related genes, genes related to production of useful substances (such as drugs, enzymes, pigments and aromatic components), genes related to yield (number or sizes) or flowering, insect resistance related genes, and genes related to resistance to environmental stresses (such as low temperature, high temperature, dryness, salt, photodamage or ultraviolet rays).

### · Cas protein:

As used herein, "Cas protein" refers to a protein belonging to the Cas protein family constituting part of the adaptive immune system which provides acquired resistance in bacteria and archaebacteria against invading foreign nucleic acid, being a target-specific endonuclease that recognizes a PAM sequence and cleaves double-stranded DNA either upstream or downstream from it. Cas proteins are RNA-guided nucleases (RGN), and together with guide RNA (gRNA) they constitute the CRISPR/Cas system. By introducing or constructing the CRISPR/Cas system in target cells it is possible for the guide RNA to bind to the target site in the genome and for the Cas protein recruited to the binding site to cleave the DNA at the target site.

The Cas protein used for the invention recognizes a specific PAM (protospacer adjacent motif) that includes 5'-NNACNN-3' (SEQ ID NO: 1). More preferably it recognizes a specific PAM sequence including 5'-NNACGN-3' (SEQ ID NO: 2) or 5'-NNACAN-3' (SEQ ID NO: 3).

The Cas protein used for the invention may be any type of Cas protein so long as it recognizes the specific PAM sequence, with no limitation on the type, although Cas9 is preferred.

The Cas protein used for the invention may be a natural protein, but it may also be a recombinant protein.

The source of the Cas protein used for the invention is not limited but is preferably a Cas protein from a bacterium belonging to *Abyssicoccus,* and more preferably AalCas9, as a Cas9 derived from *Abyssicoccus albus.* The amino acid sequence of the wild type AalCas9 is listed as SEQ ID NO: 4.

When AalCas9 is used for the invention, it may be the wild type AalCas9 having the amino acid sequence listed as SEQ ID NO: 4, and so long as its activity (for example, the endonuclease activity described below) is not impaired, it may be mutant AalCas9 having one or more mutations in the amino acid sequence listed as SEQ ID NO: 4. Specifically, such a mutant AalCas9 is preferably a protein having the amino acid sequence of the wild type AalCas9 listed as SEQ ID NO: 4 with a substitution, deletion, addition or insertion of one or more amino acids, while also exhibiting equal or superior activity compared to AalCas9 protein (for example, the endonuclease activity described below). The mutant AalCas9 protein preferably has an amino acid sequence with at least 80%, at least 85%, at least 90%, at least 92%, at least 95%, at least 96%, at least 97%, at least 98% or at least 99% sequence similarity (preferably sequence identity) with the parent wild type AalCas9 amino acid sequence listed as SEQ ID NO: 4.

The "similarity" between two amino acid sequences is the percentage of equal amino acid residues or similar amino acid residues (similar in physicochemical properties) appearing at corresponding sites when the two amino acid sequences have been aligned, while "identity" between two amino acid sequences is the percentage of equal amino acid residues appearing at corresponding sites when the two amino acid sequences have been aligned. The "homology" and "identity" of two amino acid sequences can be determined using BLAST (Basic Local Alignment Search Tool) program (Altschul et al., J. Mol. Biol., (1990), 215(3):403-10).

The nucleic acid coding for the Cas protein used for the invention has a nucleotide sequence corresponding to the amino acid sequence of the Cas protein, and it is not restricted so long as it is nucleic acid that is able to express the Cas protein in eukaryotic cells. The nucleic acid can be prepared as appropriate based on amino acid sequence information for the desired Cas protein. The nucleic acid may also be prepared by chemical synthesis.

Nucleic acid coding for the Cas protein to be used for the invention includes nucleic acid coding for the wild type AalCas9 of SEQ ID NO: 4, and also nucleic acid comprising a nucleotide sequence coding for a protein comprising an amino acid sequence with at least 80%, at least 85%, at least 90%, at least 95%, at least 96%, at least 97%, at least 98% or at least 99% sequence identity, with respect to the amino acid sequence of the wild type AalCas9 of SEQ ID NO: 4. As an example, SEQ ID NO: 5 represents the nucleotide sequence of the wild type AalCas9 gene coding for the wild type AalCas9 of SEQ ID NO: 4, in *Abyssicoccus albus.*

The nucleic acid coding for the Cas protein to be used for the invention may also be codon optimized for the translation system of the eukaryotic cells into which the nucleic acid is to be introduced For example, the nucleotide sequence of the wild type AalCas9 gene codon optimized for *E. coli* is listed as SEQ ID NO: 6, the nucleotide sequence codon optimized for humans is listed as SEQ ID NO: 7, and the nucleotide sequence codon optimized for *Arabidopsis thaliana* is listed as SEQ ID NO: 8.

The nucleic acid coding for the Cas protein to be used for the invention may be nucleic acid comprising any of the aforementioned nucleotide sequences, such as a nucleotide sequence having at least 80%, at least 85%, at least 90%, at least 95%, at least 96%, at least 97%, at least 98% or at least 99% sequence identity with the nucleotide sequences listed as SEQ ID NO: 5, SEQ ID NO: 6, SEQ ID NO: 7 or SEQ ID NO: 8, for example.

The nucleic acid coding for the Cas protein to be used for the invention may include a nuclear localization signal (NLS) sequence for transport of a protein or nucleic acid into the nucleus by nuclear transport in eukaryotic cells. The NLS may be added only to either one of the 5'-end or 3'-end of the nucleic acid coding for the Cas protein, or it may be added to both the 5'-end and 3'-end.

The nucleic acid coding for the Cas protein to be used for the invention may also be incorporated into a vector as an expression cassette in functional linkage with a regulatory sequence such as a promoter or terminator.

### · Endonuclease activity:

The Cas protein used for the invention preferably has endonuclease activity. The term "endonuclease" as used herein means an enzyme that cleaves a nucleotide strand in its middle region. The Cas9 protein having endonuclease activity to be used for the invention therefore preferably is guided by guide RNA and has enzyme activity to cleave the target DNA strand in its middle region.

The Cas protein used for the invention is not limited but has cleaving or modifying activity for its target DNA, at a temperature in the range of 10°C to 80°C, for example, preferably a temperature in the range of 20°C to 50°C, and more preferably a temperature in the range of 25°C to 40°C.

### · PAM sequence:

The Cas protein used for the invention recognizes a specific PAM sequence. As used herein, "PAM sequence" (protospacer adjacent motif) sequence) refers to a sequence that is present in the target double-strand polynucleotide and can be recognized by Cas9 protein. The length and nucleotide sequence of the PAM sequence that can be recognized by Cas protein will differ depending on the type of Cas protein.

As mentioned above, the Cas protein used for the invention recognizes a specific PAM sequence comprising 5'-NNACNN-3' (SEQ ID NO: 1). More preferably it recognizes a specific PAM sequence including 5'-NNACGN-3' (SEQ ID NO: 2) or 5'-NNACAN-3' (SEQ ID NO: 3).

### · Guide RNA:

The guide RNA is RNA having the function of guiding the site-specific DNA modifying protein, such as Cas protein, to the target site of the target nucleic acid in the genome. The site-specific DNA modifying protein normally binds with the guide RNA to form a ribonucleoprotein (RNP). By targeting of the guide RNA to the target site on the genome and cleavage of DNA at the target site of the genome by the site-specific DNA modifying protein, such as Cas protein, it is possible to change or modify the DNA sequence at the target site of the genome.

The structure of the guide RNA is usually selected as appropriate for the type of site-specific DNA modifying protein. For example, when Cas protein is used as the site-specific DNA modifying protein, the guide RNA will usually include a crRNA (CRISPR RNA) sequence that is involved in activation of the CRISPR/Cas system, and a tracrRNA (*trans-*activating crRNA) sequence that binds to the target site on the genome. In this case the guide RNA may be single-stranded RNA (sgRNA) including the crRNA sequence and tracrRNA sequence, or it may be an RNA complex obtained by complementary binding of RNA comprising the crRNA sequence and RNA comprising the tracrRNA sequence.

The crRNA sequence includes a repeat sequence and a spacer sequence. The spacer sequence is a sequence that can form a double strand with a complementary strand or essentially complementary strand to the target DNA sequence (a sequence that does not completely match the target sequence but can still bind to the target sequence). By including such a spacer sequence, the crRNA sequence allows targeted recognition by the CRISPR/Cas system. The length of the spacer sequence is not limited but is preferably a nucleotide length of 15 to 30, a nucleotide length of 18 to 25, a nucleotide length of 19 to 23 or a nucleotide length of 20 to 22, and especially 20 nucleotides, 21 nucleotides or 22 nucleotides, for example. The length of the crRNA sequence is not restricted, but the sequence may include a stem-loop with a base length of about 12 to 36, for example.

The tracrRNA sequence is a sequence that includes a highly repeating region and one, two or more (preferably two or more) consecutive hairpin structures allowing formation of multiple stem-loops. The length of the tracrRNA sequence is not restricted, but the sequence may have a base length of about 50 to 100, for example.

The structure of the guide RNA to be used for the invention is not particularly restricted so long as it can hybridize with the target DNA sequence in the genome while forming a complex with Cas protein, and is able to direct sequence-specific binding of the complex to the target DNA sequence. The design may be as appropriate for the Cas protein and the PAM sequence recognized by it according to the invention, as well as the target DNA sequence in the eukaryotic cell genome that is to be modified. Many designs are currently known for guide RNA (examples including International Patent Publication No. WO2013/142578, No. 2013/176772 and 2014/093595), and a person skilled in the art may implement any additional modifications to the invention with appropriate reference to current knowledge.

When using nucleic acid for the DNA coding for the guide RNA, its structure is not particularly restricted. DNA may be designed as appropriate depending on the sequence and construction of the guide RNA used for the invention as well as the type of eukaryotic cells to be modified. When using DNA coding for guide RNA including a crRNA sequence and tracrRNA sequence, in particular, independent molecules of DNA coding for the crRNA sequence and DNA coding for the tracrRNA sequence may be prepared, or a single DNA molecule coding for both the crRNA sequence and tracrRNA sequence may be used.

The nucleic acid for DNA coding for the guide RNA may be an independent molecule from the nucleic acid coding for the Cas protein, or it may be integral with the molecule of nucleic acid coding for the Cas protein. Nucleic acid coding for the guide RNA alone, or in tandem with nucleic acid coding for the Cas protein, may also be incorporated into a vector as an expression cassette in functional linkage with a regulatory sequence such as a promoter or terminator.

### · Target DNA sequence:

The target DNA sequence to be modified in a site-specific manner in a CRISPR/Cas system is generally not restricted, and it may be a double-stranded DNA sequence, a single-stranded RNA sequence or a single-stranded DNA sequence. According to the invention, however, the target sequence is a double-stranded DNA sequence in a eukaryotic cell genome. For most cases the target DNA sequence selected is a DNA sequence situated in proximity to the PAM sequence recognized by the Cas protein, among DNA sequences in the genome.

### · Genome editing procedure:

The method of the invention is carried out by introducing the Cas protein or nucleic acid encoding it, and the guide RNA or nucleic acid encoding it, into eukaryotic cells that have in their genome the target DNA sequence to be modified in a site-specific manner. This allows the guide RNA to bind to the target DNA in the eukaryotic cell genome, so that Cas protein recruited to the binding site can then cleave the eukaryotic cell genome at the target DNA site, modifying the genome in a site-specific manner.

The method for introducing the Cas protein or the nucleic acid encoding it and the guide RNA or the nucleic acid encoding it into eukaryotic cells is not particularly restricted. Examples include, but are not limited to, various known methods in the technical field such as methods using electroporation, liposomes, viral vectors, nanoparticles, whiskers crystals or PTD (protein transfer domain) fusion proteins, as well as the particle gun method and *Agrobacterium* method.

When the Cas protein or the nucleic acid encoding it and the guide RNA or the nucleic acid encoding it has been introduced into eukaryotic cells, the cells may be cultured under conditions suitable for cell growth. The culturing conditions may be culturing conditions suited for the cell species, and may be determined based on cell culture technology known to those skilled in the art.

### · Other:

The genome editing method of the invention has been explained above, but the invention also encompasses genome editing means used in the genome editing method, such as the Cas protein or the nucleic acid encoding it and the guide RNA or the nucleic acid encoding it.

One aspect of the invention provides a composition for genome editing and a genome editing kit to be used in the genome editing method of the invention. The composition for genome editing is a composition comprising at least the Cas protein or the nucleic acid encoding it, and optionally the guide RNA or the nucleic acid encoding it. The genome editing kit is a kit comprising at least a container containing the Cas protein or the nucleic acid encoding it, and optionally a container containing the guide RNA or the nucleic acid encoding it, with instructions describing the method for their use. The Cas protein or the nucleic acid encoding it and the guide RNA or the nucleic acid encoding it were described in detail above.

Much is currently known regarding genome editing using a CRISPR/Cas system (examples including International Patent Publication No. WO2013/142578, No. 2013/176772 and 2014/093595), and a person skilled in the art may implement any additional modifications to the invention with appropriate reference to current knowledge. As mentioned above, all of these patent documents are incorporated herein in their entirety by reference, for all purposes.

### EXAMPLES

The present invention will now be explained in further detail by Examples, with the understanding that these are merely provided for convenience of explanation and are not intended to limit the invention in any sense. The letter "N" represents any nucleotide selected from the group consisting of adenine, cytosine, thymine and guanine.

### [Example 1] Preparation of Cas9 protein from Abyssicoccus albus

### (1) Preparation of Cas9 gene from Abyssicoccus albus

The DNA sequence listed as SEQ ID NO: 5 coding for the Cas protein was artificially synthesized based on information for the *Abyssicoccus* albus-derived amino acid sequence listed as SEQ ID NO: 4. The DNA sequence can be found in a well-known database such as GenBank of the NCBI (National Center for Biotechnology Information). There were each synthesized a DNA sequence optimized for *E. coli* codons (SEQ ID NO: 6), a DNA sequence optimized for human codons (SEQ ID NO: 7) and a DNA sequence optimized for *Arabidopsis thaliana* (SEQ ID NO: 8), from the *Abyssicoccus* albus-derived Cas9 gene. The Sanger method or a next-generation sequencing method was used to determine the nucleotide sequence. The AalCas9 protein was relatively small (1059 amino acids) compared to other Cas9 orthologs such as SpyCas9 (1368 amino acids).

### (2) Assembly of vector for expression in E. coli

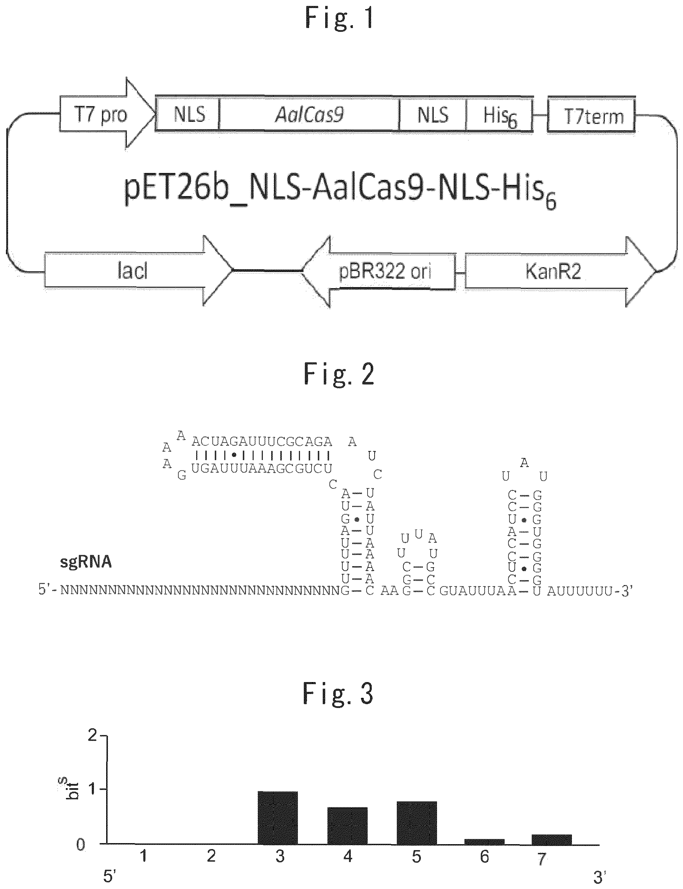

Using the procedure described in detail below, a publicly known N-terminal nuclear localization signal sequence, C-terminal nuclear localization signal sequence and His-tag were added to the AalCas9 gene of SEQ ID NO: 6 which was optimized for *E. coli* codons, to construct a vector for production of AalCas9 incorporated into pET vector. The structure of the vector is shown schematically in Fig. 1.

First, the primer 1 (SEQ ID NO: 9) and primer 2 (SEQ ID NO: 10) were used for linear DNA amplification by PCR reaction, with pET26b vector as template.

· Primer 1 (SEQ ID NO: 9): 
5'-
TCCTCCAGAGACTTTCCGCTTCTTCTTTGGGGCTTTATGATTCATATGTATATCTCC
 TTCTTAAAG-3'
· Primer 2 (SEQ ID NO: 10):

5'-
TCTGGCGGCTCAAAAAGAACCGCCGACGGCAGCGAATTCGAGCCCAAGAAGAA
GAGGAAAGTCCTCGAGCACCACCAC-3'

Separately, DNA fragments were each prepared by adding oligonucleotide A (N-terminal nuclear localization signal sequence, SEQ ID NO: 11) and oligonucleotide B (C-terminal nuclear localization signal sequence, SEQ ID NO: 12) to the 5'-end and 3'-end of the AalCas9 gene (SEQ ID NO: 6) which had been codon optimized for *E. coli.*

· Oligonucleotide A (N-terminal nuclear localization signal sequence, SEQ ID NO: 11):
   5'-CGGAAAGTCTCTGGAGGA-3'
· Oligonucleotide B (C-terminal nuclear localization signal sequence, SEQ ID NO: 12):
   5'-GCGCTAAGAACGAGCGCG-3'

The obtained DNA fragments comprising the AalCas9 gene which were codon optimized for *E. coli* were mixed with the previously obtained PCR amplification product of the pET vector, and the DNA fragments were formed into a circular plasmid using NEBuilder HiFi DNA Assembly Master Mix.

*E. coli* DH5a was transformed with the obtained circular plasmid and cultured on an LB agar plate containing 50 µg/mL kanamycin to select transformants. Several colonies were picked up and the target circular plasmids (pET-AalCas9) taken from them were prepared as vectors for AalCas9 production.

### (3) Preparation of AalCas9:

The obtained plasmid vector pET-AalCas9 was introduced into *E. coli* ROSETTA2(DE3)pLYSS by a common method. The obtained recombinant strain was cultured in LB medium containing 50 µg/mL kanamycin, and at the culturing point where the OD value reached 0.8, isopropyl-β-thiogalactopyranoside (Isopropyl β-D-1-thiogalactopyranoside: IPTG) was added (final concentration: 0.25 mM) as an expression inducing agent, and culturing was continued for 20 hours at 20°C. After culturing, the *E. coli* cells were collected by centrifugal separation (8,000 g, 10 min). The collected cells were suspended in buffer A (50 mM Tris-HCl, pH 8.0, 500 mM NaCl, 5 mM MgCl₂) and subjected to ultrasonic disruption. The supernatant was recovered by centrifugal separation (12,000 g, 30 min), and after purification with a Ni-affinity column equilibrated with buffer A and subsequently with a Heparin affinity column, it was purified with a gel filtration column equilibrated with buffer B (20 mM Tris-HCl, pH 7.5, 300 mM NaCl, 1 mM MgCl₂, 10% glycerol). The purified protein was concentrated using a centrifugal ultrafiltration filter unit (Merck Millipore) to obtained AalCas9. The obtained AalCas9 was stored at -80°C.

### [Example 2] Identification of PAM sequence:

### (1) Creation of plasmid library:

A plasmid library was created by the following procedure for identification of the PAM sequence of the AalCas9 gene.

First, a fragment obtained by cleaving commercially available pUC18 with restriction enzymes BamH1 and EcoR1 was mixed with a double-stranded DNA fragment annealed with the following oligonucleotide C (SEQ ID NO: 13), which was phosphorylated at the 5'-end and included part of the publicly known rice-derived PDS (phytoene desaturase) gene sequence (5'-GTTGGTCTTTGCTCCTGCAG-3'), and oligonucleotide D (SEQ ID NO: 14), and they were ligated using Ligation mix (Takara Corp.).

· Oligonucleotide C (SEQ ID NO: 13):
   5'-pGATCGTTGGTCTTTGCTCCTGCAGAGG-3'
· Oligonucleotide D (SEQ ID NO: 14):
   5'-pAATTCCTCTGCAGGAGCAAAGACCAAC-3'

The obtained ligation solution was used for transformation of *E. coli* DH5α by a common method, and after screening on an LB agar plate containing 100 µg/mL ampicillin, a plasmid was purified from a clone having the target recombinant plasmid (pUC18-OsPDS).

The purified pUC18-OsPDS was used as template for a first PCR reaction using the following primer 3 (SEQ ID NO: 15) and primer 4 (SEQ ID NO: 16), to obtain a first PCR amplification product containing the amplified DNA fragment.

· Primer 3 (SEQ ID NO: 15)
   5'-ACATCTGACGCTGCCGACGACAAGCTTGGCACTGGCCGTCG-3'
· Primer 4 (SEQ ID NO: 16)
   5'-CCGGAAGCATAAAGTGTAAAGC-3'

The purified pUC18-OsPDS was used as template in the same manner for a second PCR reaction using the following primer 5 (SEQ ID NO: 17) and primer 6 (SEQ ID NO: 18) containing the adapter sequence used for NGS analysis, to obtain a second PCR amplification product containing the amplified DNA fragment.

· Primer 5 (SEQ ID NO: 17) 
5'-
CTTGTCGTCGGCAGCGTCAGATGTGTATAAGAGACAGCATGCCTGCAGGTCGAC
TCGAGAGG-3'
· Primer 6 (SEQ ID NO: 18)

5'-
ACACTTTATGCTTCCGGGTCTCGTGGGCTCGGAGATGTGTATAAGAGACAGCTCG
TATGTTGTGTGGAATTG-3'

The obtained first and second PCR amplification products were mixed and the DNA fragments were formed into a circular plasmid using NEBuilder HiFi DNA Assembly Master Mix. *E. coli* DH5α was transformed with the circularized plasmid, and after screening on an LB agar plate containing 100 µg/mL ampicillin, a plasmid was purified from a clone having the target recombinant plasmid (pUC18-NGS-OsPDS).

After mixing oligonucleotide E (SEQ ID NO: 19) and oligonucleotide F (SEQ ID NO: 20) with the obtained plasmid pUC18-NGS-OsPDS, reverse transcriptase ReverTra Ace (Toyobo) was used to prepare a double-stranded DNA fragment. The oligonucleotide F included a random sequence consisting of 7 nucleotides N.

· Oligonucleotide E (SEQ ID NO: 19):
   5'-GGTCTTTGCTCCTGCAG-3'
· Oligonucleotide F (SEQ ID NO: 20):
   5'-AGCTATGACCATGATTACGAATTCNNNNNNNCTGCAGGAGCAAAGACC-3'

The purified pUC18-NGS-OsPDS was used as template for a PCR reaction using the following primer 7 (SEQ ID NO: 21) and primer 8 (SEQ ID NO: 22), to obtain a PCR amplification product containing the amplified DNA fragment.

· Primer 7 (SEQ ID NO: 21):
   5'-CTGCAGGAGCAAAGACC-3'
· Primer 8 (SEQ ID NO: 22):
   5'-GTAATCATGGTCATAGCTGTTTCC-3'

The PCR amplification product containing the obtained DNA fragment was mixed with the prepared double-stranded DNA fragment containing the random sequence, and the DNA fragments were formed into a circular plasmid using NEBuilder HiFi DNA Assembly Master Mix.

*E. coli* DH5α was transformed with the obtained circularized plasmid, and after screening on an LB agar plate containing 100 µg/mL ampicillin, approximately 100,000 colonies having the target recombinant plasmid were collected and cultured, and a pUC18-NGS-OsPDS-7N series plasmid library was constructed using NucleoBond EXtra Midi Plus (Nippon Genetics Co, Ltd.).

### (2) Preparation of RNP for cleavage of plasmid library

AalCas9 sgRNA (SEQ ID NO: 23) for cleavage of plasmid library pUC18-NGS-OsPDS-7N was prepared by *in vitro* transcription using a Guide-it^{™} sgRNA In Vitro Transcription Kit (Takara Bio, Inc.). The structure of AalCas9 sgRNA is shown in Fig. 2, and the nucleotide sequence is listed as SEQ ID NO: 23. The poly-N sequence at the 5'-end is omitted in SEQ ID NO: 23. The AalCas9 sgRNA was diluted with RNase-free Cas9 buffer (20 mM HEPES-Na, pH 7.5, 100 mM NaCl, 5 mM MgCl₂, 0.1 mM EDTA) to 2.5 µM AalCas9 and 12.5 µM sgRNA, and the mixture was then allowed to stand at 25°C for 20 minutes to form a complex, thereby preparing a first RNP solution.

### · AalCas9 sgRNA (SEQ ID NO: 23)

5'-
GUUUUAGUACUCUGCGAAAUUUAGUGAAAACUAGAUUUCGCAGAAUCUAUU
AAAACAAGGCUUUAUGCCGUAUUUAACUCCAUCCUAUGGGUGGGGUAUUUU
UU-3'

### (3) Identification of PAM sequence by NGS analysis:

The plasmid library and Cas9 buffer were added to the first RNP solution prepared in (2) above to a concentration of 20 nM, and enzyme reaction was conducted for 2 hours at 30°C. After adding EDTA to the reaction mixture to a concentration of 60 mM, the mixture was incubated for 5 minutes at 60°C and then subjected to agarose electrophoresis to purify the uncut plasmid.

The purified uncut plasmid was used as template for PCR reaction using the following primer 9 (SEQ ID NO: 24) and primer 10 (SEQ ID NO: 25), to obtain a PCR amplification product containing an approximately 370 bp double-stranded DNA fragment including a random sequence.

· Primer 9 (SEQ ID NO: 24)
   5'-GCGATCGGTGCGGGCCTCTTCGCTATTACGC-3'
· Primer 10 (SEQ ID NO: 25)
   5'-ATTAGGCACCCCAGGCTTTACACTTTATG-3'

The PCR amplification product containing the double-stranded DNA fragment was diluted with Cas9 buffer to a concentration of 200 nM, and then the first RNP solution of (2) above was added and enzyme reaction was conducted for 30 minutes at 37°C. The reaction mixture was subjected to agarose electrophoresis in the same manner to purify the uncut double-stranded DNA fragment. The purified double-stranded DNA fragment was used as a DNA sequence analysis sample using a next-generation sequencer (NGS).

The purified double-stranded DNA fragment and the non-RNP-treated plasmid library were used for NGS analysis using Hiseq (Illumina) and the sequence in the random region was detected. The PAM sequence of AalCas9 was identified as NNACGN, by comparing the sequence in the random region of the purified double-stranded DNA fragment with the sequence in the non-RNP-treated plasmid library, and extracting common sequences from among those whose detection frequency was reduced to 1/10 in the purified double-stranded DNA fragment (Fig. 3).

### [Example 3] DNA cleavage activity measurement in vitro:

### (1) Preparation of substrate DNA:

A PCR reaction was carried out with the pUC18-NGS-OsPDS-7N prepared in Example 2 as template, using a primer set comprising primer 11 (SEQ ID NO: 26) and primer 12 (SEQ ID NO: 27) shown below, including the AalCas9 PAM sequence, or a primer set comprising primer 13 (SEQ ID NO: 28) and primer 12 (SEQ ID NO: 27) shown below, including the publicly known SpyCas9 PAM sequence, to prepare PCR amplification products each containing an approximately 120 bp DNA fragment. The prepared DNA fragments were purified using a FastGene Gel/PCR extraction kit (Nippon Genetics Co, Ltd.), and used as substrates for DNA cleavage activity measurement.

### <For AalCas9>

· Primer 11 (SEQ ID NO: 26) 
5'-
CGGATAACAATTTCACACAGGAAACAGCTATGACCATGATTACGAATTCTGCGTT
ACTGCAGGAGC-3'
· Primer 12 (SEQ ID NO: 27)
   5'-GCGATCGGTGCGGGCCTCTTCGCTATTACGC-3'

### <For SpyCas9>

· Primer 13 (SEQ ID NO: 28) 
5'-
CGGATAACAATTTCACACAGGAAACAGCTATGACCATGATTACGAATTCTTTCCC
ACTGCAGGAGC-3'
· Primer 12 (SEQ ID NO: 27)
   5'-GCGATCGGTGCGGGCCTCTTCGCTATTACGC-3'

### (2) Preparation of RNP for DNA cleavage activity measurement in vitro:

AalCas9 sgRNA (SEQ ID NO: 23) for *in vitro* DNA cleavage activity measurement was prepared by *in vitro* transcription using a Guide-it^{™} sgRNA In Vitro Transcription Kit (Takara Bio, Inc.). The AalCas9 sgRNA was diluted with RNase-free Cas9 buffer (20 mM HEPES-Na, pH 7.5, 100 mM NaCl, 5 mM MgCl₂, 0.1 mM EDTA) to 2 µM AalCas9 and 2 µM sgRNA, and the mixture was then allowed to stand at 25°C for 20 minutes to form a complex, thereby preparing a second RNP solution.

### (3) DNA cleavage activity measurement in vitro:

Substrate DNA and Cas9 buffer were added to the second RNP solution of (2) above, to a concentration of 1 µM RNP and 10 nM substrate DNA. The reaction mixture was incubated at 25°C and 37°C, recovering a portion of the reaction mixture after 5 minutes, 10 minutes, 15 minutes and 30 minutes, and then EDTA was added to a concentration of 50 mM and the mixture was incubated for 5 minutes at 65°C to suspend the reaction. The uncleaved DNA and cleaved DNA in the collected reaction mixture were quantified using MultiNA (Shimadzu) to measure the DNA cleavage activity.

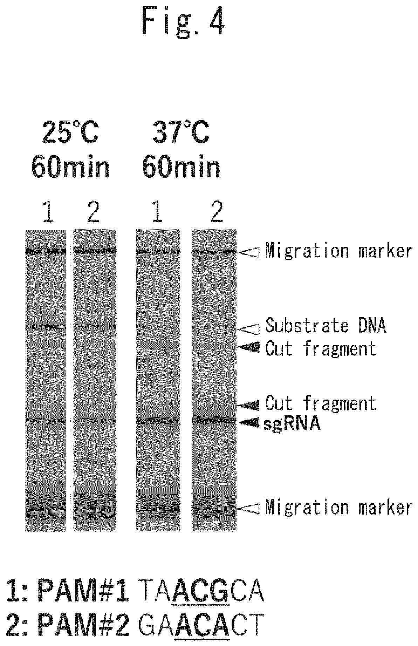

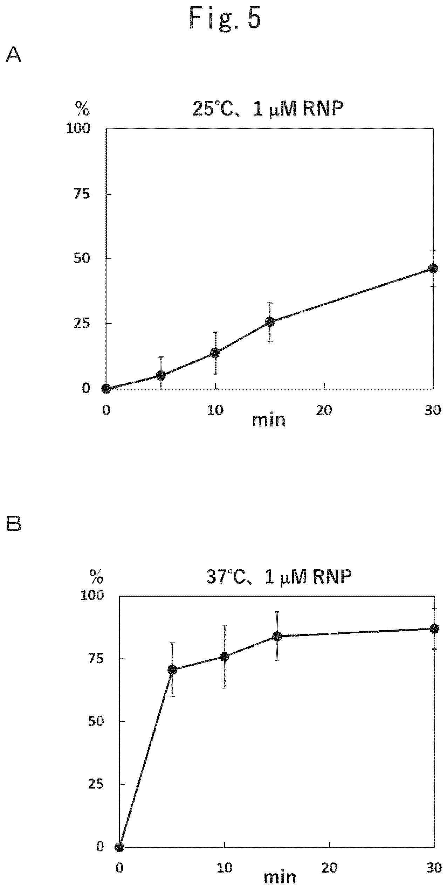

Fig. 4 is a photograph showing an example of electrophoresis results for the reaction product of an *in vitro* DNA cleavage assay. The left side of the photograph shows the results of incubating the reaction mixture for 60 minutes at 25°C, and the right side shows the results of incubating the reaction mixture for 60 minutes at 37°C. Fig. 5A and 5B are graphs showing time-dependent change in AalCas9 activity in an *in vitro* DNA cleavage assay. The graph of Fig. 5A shows the results of incubating the reaction mixture at 25°C, and the graph of Fig. 5B shows the results of incubating the reaction mixture at 37°C. Based on these results, AalCas9 was shown to cleave at least 80% of the substrate DNA after 15 minutes at 37°C, and to cleave approximately 50% of the substrate DNA after 30 minutes at 25°C.

### [Example 4] Mutagenesis of plant cells with AalCas9:

### (1) Preparation of RNP for genome editing test in Arabidopsis thaliana protoplasts

The sequence of sgRNA (SEQ ID NO: 29) including the PAM sequence ACGN in the publicly known *Arabidopsis thaliana-*derived PDS3 gene was prepared by *in vitro* transcription using a Guide-it^{™} sgRNA In Vitro Transcription Kit (Takara Bio, Inc.). The sgRNA was diluted with RNase-free Cas9 buffer (20 mM HEPES-Na, pH 7.5, 100 mM NaCl, 5 mM MgCl₂, 0.1 mM EDTA) to 10 µM AalCas9 and 20 µM sgRNA, and the mixture was then allowed to stand at 25°C for 20 minutes to form a complex, thereby preparing a third RNP solution. The poly-N sequence at the 5'-end is omitted in SEQ ID NO: 29.

### · PDS3 sgRNA (SEQ ID NO: 29)

5'-
GUAGCGAGUAAUCGUGAAGUAGAGAAACAGGGUUUUAGUACUCUGCGAAAU
UUAGUGAAAACUAGAUUUCGCAGAAUCUAUUAAAACAAGGCUUUAUGCCGU
AUUUAACUCCAUCCUAUGGGUGGGGUAUUUUUU-3'

SpyCas9 sgRNA (SEQ ID NO: 30) having the following publicly known sequence was also prepared by the same procedure, and an RNP complex was formed in the same manner as AalCas9, to prepare a fourth RNP solution. The poly-N sequence at the 5'-end is omitted in SEQ ID NO: 30.

### · SpyCas9 sgRNA (SEQ ID NO: 30)

5'-
GGACUUUUGCCAGCCAUGGUGUUUUAGAGCUAGAAAUAGCAAGUUAAAAUA
AGGCUAGUCCGUUAUCAACUUGAAAAAGUGGCACCGAGUCGGUGCUUUUU-
3'

### (2) Preparation of Arabidopsis thaliana protoplasts:

*Arabidopsis thaliana* leaves were grown for 30 days, and after stripping the epidermis to expose the mesophyll cells, the leaves were immersed in a solution containing 1.0% Cellulase Onozuka R10 (Yakult Honsha Co., Ltd.), 0.25% Macerozyme R-10 (Yakult Honsha Co., Ltd.), 10 mM mercaptoethanol, 400 mM mannitol, 20 mM KCl, 10 mM CaCl₂ and 20 mM MES (pH 5.7), and shake cultured at 22°C, 50 rpm while incubating for 1 hour, to free the protoplasts. The protoplasts were filtered with a 70 µm-pore size nylon filter and then collected in a 50 mL-volume tube, and after recovering the protoplasts by centrifugation at 100 × g for 10 minutes and discarding the supernatant, they were resuspended in buffer (W5 buffer) containing 150 mM NaCl, 125 mM CaCl₂, 5 mM KCl and 2 mM MES (pH 5.7). The resuspended protoplasts were centrifuged for 5 minutes at 100 × g and then resuspended in the same buffer. After twice washing with buffer, the cells were incubated at 4°C for 10 minutes. The incubated protoplasts were centrifuged at 100 × g for 5 minutes and then resuspended in buffer containing 400 mM mannitol, 15 mM MgCl₂ and 4 mM MES (pH 5.7). After then recovering the protoplasts by centrifugation at 100 × g for 5 minutes, the cell concentration was adjusted to 2.0 to 3.0 × 10⁵ cells/mL with buffer to obtain a protoplast suspension for transformation.

### (3) Transfer of RNP into Arabidopsis thaliana protoplasts:

After mixing 35 µL of the protoplast suspension obtained in (2) above with 10 µL of either the third or fourth RNP solution, 45 µL of a solution containing 40% (w/v) PEG4000, 200 mM mannitol and 100 mM CaCl₂ was placed in each well of a 96-well plate (roundbottom, product of Nunc Co.), and shaking was carried out at 900 rpm for 15 seconds to mix the solution. The liquid mixture was allowed to stand at room temperature for 10 minutes. After suspending the protoplasts by vigorously adding 200 µL of W5 buffer to the standing liquid mixture, they were centrifuged at 100 × g for 5 minutes, and the protoplast cells were collected at the plate bottom, discarding 200 µL of the supernatant and washing the protoplast suspension. After repeating this procedure a total of 4 times, each well was sealed with a Parafilm and allowed to stand for 18 hours at 22°C, for mutagenesis of the genomic DNA.

### (4) NGS analysis of genomic DNA:

After transfer of the RNP of (3) above, the protoplast suspension that had been allowed to stand at 22°C for 18 hours was collected in a 200 µL-volume tube and mixed with genome extraction buffer (200 mM Tris-HCl, pH 7.5, 250 mM NaCl, 25 mM EDTA, 0.5% SDS), after which it was incubated at 95°C for 10 minutes and then allowed to stand for 5 minutes on ice. The genomic DNA was recovered by isopropanol precipitation from the heat-treated protoplast suspension. A PCR reaction was carried out with the collected genomic DNA suspended in sterilized water, using a primer set comprising primer 14 (SEQ ID NO: 31) and primer 15 (SEQ ID NO: 32) shown below for AalCas9, and a primer set comprising primer 16 (SEQ ID NO: 33) and primer 17 (SEQ ID NO: 34) shown below for SpyCas9, for amplification of an approximately 300 bp DNA fragment. The obtained DNA fragment of approximately 300 bp was subjected to next-generation sequence analysis using an iSeq100 system (Illumina Corp.), to detect the presence or absence of genome editing.

### <For AalCas9>

· Primer 14 (SEQ ID NO: 31) 
5'-
TCGTCGGCAGCGTCAGATGTGTATAAGAGACAGGGAAAAGAATCATATGGTCAT
CAATTCG-3'
· Primer 15 (SEQ ID NO: 32)

5'-
GTCTCGTGGGCTCGGAGATGTGTATAAGAGACAGCGGACTTGAATTAAAGGGGC
TATAGTAG-3'

### <For SpyCas9>

· Primer 16 (SEQ ID NO: 33) 5'- 
TCGTCGGCAGCGTCAGATGTGTATAAGAGACAGGCAAATAATTAAAGTTGTTGCT
GTTGG-3'
· Primer 17 (SEQ ID NO: 34)

5'-
GTCTCGTGGGCTCGGAGATGTGTATAAGAGACAGTAGCCTACTTGCCTGCTTTTC
-3'

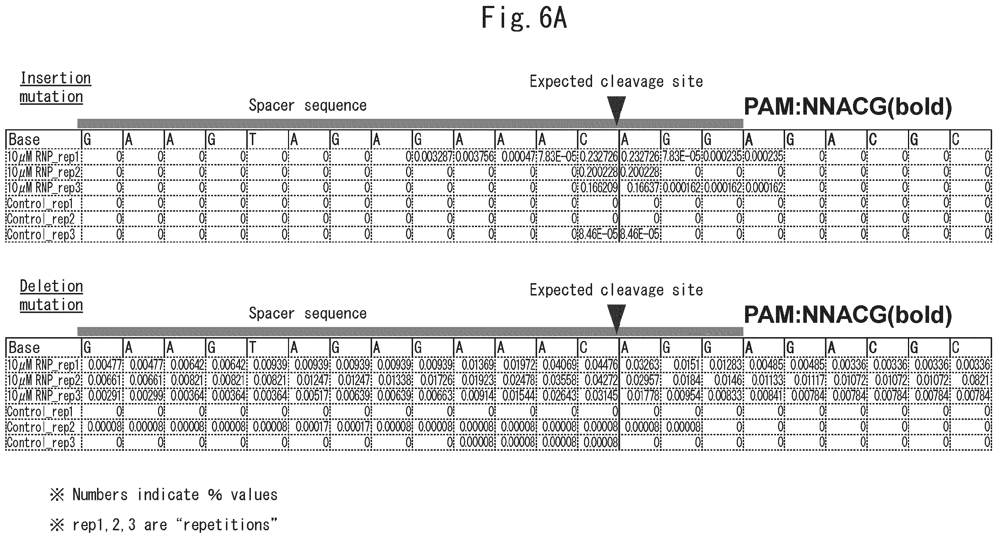

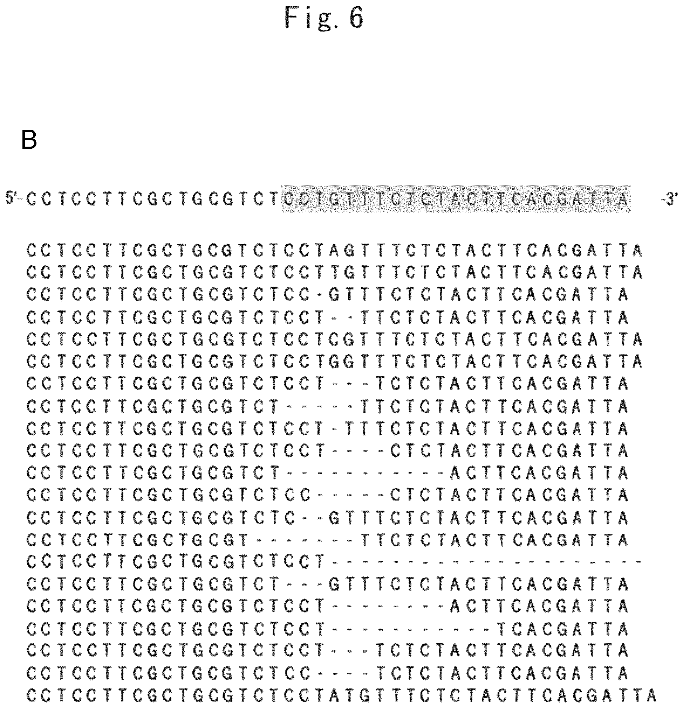

Figs. 6A and 6B show results of genomic DNA editing in *Arabidopsis thaliana* protoplast cells by AalCas9. Fig. 6A shows the editing rate for each target DNA, and Fig. 6B shows mutant DNA determined using a next-generation sequencer.

Based on these results, genomic DNA editing of *Arabidopsis thaliana* cells by AalCas9 resulted in detection of deletion and insertion in short to long nucleotide regions of the target gene. The mutation rate per cell with AalCas9 was 25.6%, which was superior compared to SpyCas9.

### [Example 5] Genome editing of Arabidopsis thaliana plant body using AalCas9 by the Agrobacterium method

### (1) Construction of binary vector

A sequence comprising an N-terminal nuclear localization signal sequence (SEQ ID NO: 35) and C-terminal nuclear localization signal sequence (SEQ ID NO: 36) added to the AalCas9 gene (SEQ ID NO: 5) optimized for human codons was incorporated by a publicly known method into a binary vector (pCA) whose expression is regulated by the RPS5a promoter sequence and the HSP terminator sequence and Pea3 terminator sequence, to construct pCA-NLS-AalCas9-NLS.

A sequence (SEQ ID NO: 37) comprising the *Arabidopsis thaliana* U6 promoter sequence added to AalCas9 sgRNA for mutagenesis of *Arabidopsis thaliana,* designed to facilitate introduction of the target sequence, was then incorporated by a publicly known method into pCA-NLS-AalCas9-NLS, to construct pCA-NLS-AalCas9-NLS-U6-Bsal-gRNA. A fragment obtained by cutting this pCA-NLS-AalCas9-NLS-U6-Bsa1-gRNA with Bsa1 was ligated with a double-stranded DNA fragment annealed with oligonucleotide G (SEQ ID NO: 38) phosphorylated at the 5'-end and including a portion of the *Arabidopsis thaliana*-derived PDS3 gene sequence (5'-TCGTGAAGTAGAGAAACAGG-3') and its complementary sequence, and oligonucleotide H (SEQ ID NO: 39).

· Oligonucleotide G (SEQ ID NO: 38):
   5'-pATTGTCGTGAAGTAGAGAAACAGG-3'
· Oligonucleotide H (SEQ ID NO: 39):
   5'-pAAACCCTGTTTCTCTACTTCACGA-3'

*E. coli* DH5α was transformed with the obtained ligation solution, and after screening on an LB agar plate containing 50 µg/mL spectinomycin, a plasmid was purified from a clone having the target recombinant plasmid (pCA-NLS-AalCas9-NLS-U6-PDS3-gRNA).

### (2) Transformation of Arabidopsis thaliana by pCA-NLS-AalCas9-NLS-U6-PDS3-gRN using floral dip method

The pCA-NLS-AalCas9-NLS-U6-PDS3-gRNA constructed in (1) above was introduced into publicly known *Agrobacterium tumefaciens* GV3101 (C58C1 Rifr)pMP90 (Gmr) (see Koncz et al, Mol. Gen. Genet., (1986), 204:383-396), by electroporation. The obtained cells were cultured for 2 days in LB medium containing 20 mL antibiotics (50 µg/mL spectinomycin, 25 µg/mL gentamicin and 50 µg/mL rifampicillin), and the cells were recovered and suspended in 30 mL of infiltration medium.

The *Arabidopsis thaliana* wild strain Col-0 was cultivated for 45 to 60 days and allowed to bloom, and after cutting off the main stems it was further cultivated for 10 days and used for transformation. The transformation was carried out by infecting *Arabidopsis thaliana* by dropwise addition of infiltration medium which had suspended the cells into which the transformation vector had been introduced, and after normal raising, the T1 seeds were harvested. The T1 seeds were then sterilized for 7 minutes with a solution of 50% bleach and 0.02% Triton X-100, rinsed 3 times with sterilized water, and seeded in MS selection medium containing 25 µg/mL hygromycin.

### (3) NGS analysis of genomic DNA

Leaves were collected from the *Arabidopsis thaliana* individuals selected after 2 weeks in (2) above and were mixed with genome extraction buffer (200 mM Tris-HCl (pH 7.5), 250 mM NaCl, 25 mM EDTA, 0.5% SDS), after which they were crushed with a ShakeMaster (Bio Medical Science Inc.) and the genomic DNA was recovered by isopropanol precipitation. The recovered genomic DNA was suspended in sterilized water and a DNA fragment of approximately 300 bp amplified using the following primer set consisting of primer 14 (SEQ ID NO: 31) and primer 15 (SEQ ID NO: 32) was subjected to NGS analysis with an iSeq100 system (Illumina), for detection of genome editing.

· Primer 14 (SEQ ID NO: 31) 
5'-
TCGTCGGCAGCGTCAGATGTGTATAAGAGACAGGGAAAAGAATCATATGGTCAT
CAATTCG-3'
· Primer 15 (SEQ ID NO: 32)

5'-
GTCTCGTGGGCTCGGAGATGTGTATAAGAGACAGCGGACTTGAATTAAAGGGGC
TATAGTAG-3'

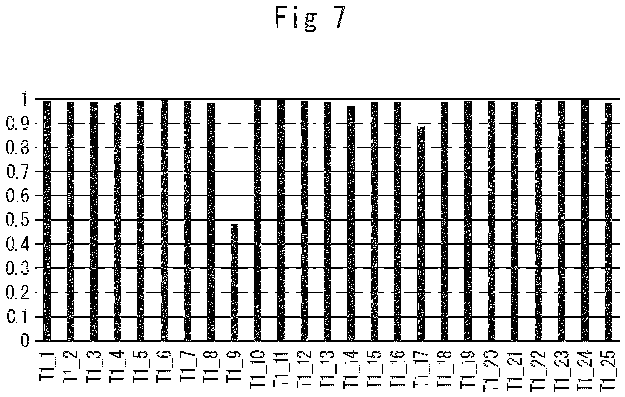

Fig. 7 is a graph showing the results of genomic DNA analysis of leaves of *Arabidopsis thaliana* T1 individuals transformed with pCA-NLS-AalCas9-NLS-U6-PDS3-gRNA by the procedure described above, with the editing efficiency of each nucleotide of the PDS3 gene detected for each T1 individual No. (maximum value = 1). Based on these results, genomic DNA editing of *Arabidopsis thaliana* plant bodies by AalCas9 resulted in highly efficient confirmation of genome edited individuals with homozygous deletion and insertion of nucleotides in the target gene.

### [Example 6] Genome editing of human cultured cells using AalCas9

### (1) Vector assembly

A sequence comprising an N-terminal nuclear localization signal sequence (SEQ ID NO: 35) and C-terminal nuclear localization signal sequence (SEQ ID NO: 36) added to the AalCas9 gene (SEQ ID NO: 5) optimized for human codons was incorporated by a publicly known method into a plasmid (pCMV) whose expression is regulated by the CMV promoter sequence and BGH polyA sequence, to construct pCMV-NLS-AalCas9-NLS.

A sequence (SEQ ID NO: 40) comprising the human U6 promoter sequence added to AalCas9 sgRNA designed to facilitate introduction of the target sequence, was then incorporated by a publicly known method into pCMV-NLS-AalCas9-NLS, to construct pCMV-NLS-AalCas9-NLS-U6-Bbs1-gRNA. A fragment obtained by cutting the constructed pCMV-NLS-AalCas9-NLS-U6-Bbs1-gRNA with Bbs1 was ligated with a double-stranded DNA fragment including a portion of the human-derived AAVS 1 gene sequence and annealed with oligonucleotide I (SEQ ID NO: 41) and oligonucleotide J (SEQ ID NO: 42) which were phosphorylated at the 5'-end.

· Oligonucleotide I (SEQ ID NO: 41):
   5'-pCACCCTGCAGCACCAGGATCAGTG-3'
· Oligonucleotide J (SEQ ID NO: 42):
   5'-pAAACCACTGATCCTGGTGCTGCAG-3'

*E. coli* DH5α was transformed with the obtained ligation solution, and after screening on an LB agar plate containing 100 µg/mL ampicillin, a plasmid was purified from a clone having the target recombinant plasmid (pCMV-NLS-AalCas9-NLS-U6-hAAVS1-gRNA).

### (2) Introduction of AalCas9 expression vector into human cultured cells

The pCMV-NLS-AalCas9-NLS-U6-hAAVS1-gRNA prepared in (1) above was mixed with Lipofectamine 3000 (Thermo Fisher Scientific) and used for transfection into HEK293FT cells (~10⁴ cells) that had been cultured in a 96-well plate, and then cultured for 3 days. The transfected HEK293FT cells were collected and disrupted by mixture with 0.1 N NaOH.

### (3) NGS analysis of genomic DNA

The cultured HEK293FT cells were collected and mixed with 0.1 N NaOH to disrupt the cells, and a DNA fragment of approximately 300 bp amplified using part of the disrupted cells and a primer set consisting of primer 18 (SEQ ID NO: 43) and primer 19 (SEQ ID NO: 44) was subjected to NGS analysis with an iSeq100 system (Illumina), and genome editing was detected based on deletion of the target sequence.

· Primer 18 (SEQ ID NO: 43): 
5'-
TCGTCGGCAGCGTCAGATGTGTATAAGAGACAGAAACTGCTTCTCCTCTTGGGA-
3'
· Primer 19 (SEQ ID NO: 44):

5'-
GTCTCGTGGGCTCGGAGATGTGTATAAGAGACAGCAGATTCCTTATCTGGTGACA

CA-3'

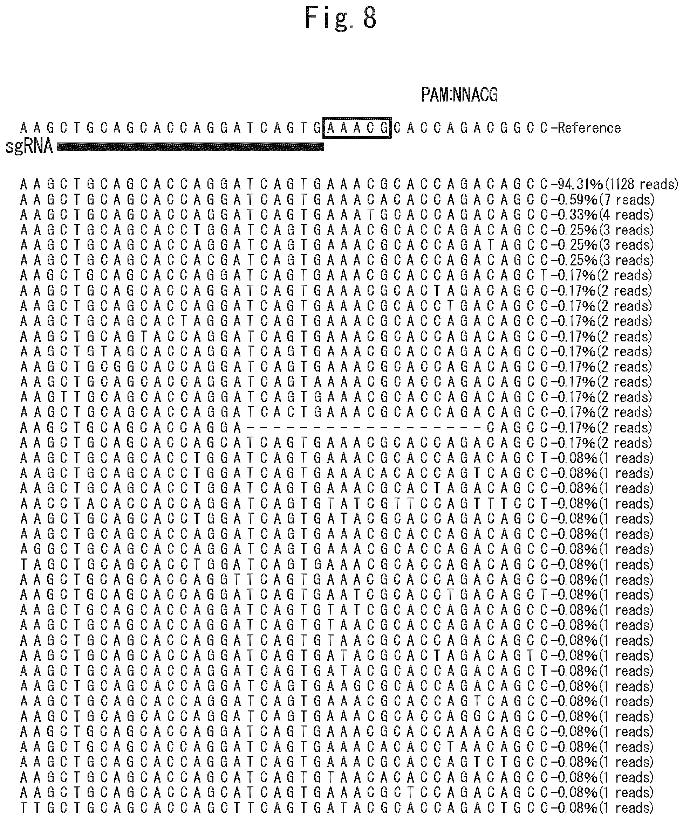

Fig. 8 is a diagram showing the results of comparing the genome DNA of HEK293FT cells transformed with pCMV-NLS-AalCas9-NLS-U6-hAAVS1-gRNA by the procedure described above, with the original human genome hAAVS1 gene sequence, and the detected genomic DNA sequences (number of reads) and the editing ratio (= number of DNA with detected editing/total number of DNA analyzed). Based on these results, genomic DNA editing of human cultured cells by AalCas9 resulted in detection of nucleotide deletion at the gRNA binding site of the target gene.

### INDUSTRIAL APPLICABILITY

The *Abyssicoccus* albus-derived Cas9 of the invention has a wide range of usefulness in a variety of industrial fields which deal with genomic variants, such as agriculture, the pharmaceutical industry, and the enzyme industry.

[Sequence Listing]

## Claims

1. A method for modifying a target DNA sequence in a genome of eukaryotic cells in a site-specific manner, wherein the method comprises introducing into the eukaryotic cells:
(1) Cas protein recognizing a PAM (protospacer adjacent motif) sequence containing the nucleotide sequence 5'-NNACNN-3' listed as SEQ ID NO: 1 (where each "N" represents any one nucleotide independently selected from among adenine, cytosine, thymine and guanine), or nucleic acid coding for the Cas protein, and
(2) guide RNA that can hybridize to the target DNA sequence in the genome while also forming a complex with the Cas protein, and that can direct sequence-specific binding of the complex to the target DNA sequence, or nucleic acid coding for the guide RNA,
to modify the eukaryotic cell genome at the target DNA sequence.

2. The method according to claim 1, wherein the Cas protein is derived from an *Abyssicoccus* bacterium, and preferably *Abyssicoccus albus.*

3. The method according to claim 1 or 2, wherein the Cas protein includes a nuclear localization signal (NLS).

4. The method according to any one of claims 1 to 3, wherein the Cas protein includes an amino acid sequence with at least 80%, at least 85%, at least 90%, at least 92%, at least 95%, at least 96%, at least 97%, at least 98% or at least 99% sequence similarity to the amino acid sequence listed as SEQ ID NO: 4.

5. The method according to any one of claims 1 to 4, wherein the Cas protein has endonuclease activity.

6. The method according to any one of claims 1 to 5, wherein the PAM sequence includes the nucleotide sequence 5'-NNACGN-3' listed as SEQ ID NO: 2 or the nucleotide sequence 5'-NNACAN-3' listed as SEQ ID NO: 3.

7. The method according to any one of claims 1 to 6, wherein the guide RNA is single-stranded guide RNA (sgRNA), and also includes crRNA and tracrRNA.

8. The method according to claim 7, wherein the crRNA includes a spacer sequence with a nucleotide length of 15 to 30, which can form a double strand with a complementary strand of the target DNA.

9. The method according to claim 7 or 8, wherein the crRNA includes a stem-loop with a nucleotide length of 12 to 36.

10. The method according to any one of claims 1 to 9, wherein the eukaryotic cells are animal cells, plant cells or microbial cells.

11. The method according to any one of claims 1 to 10, wherein the nucleotide sequence of the nucleic acid coding for the Cas protein is codon-optimized for expression in animal cells, plant cells or microbial cells.

12. A composition for genome editing to be used in the method according to any one of claims 1 to 11, the composition comprising the Cas protein or nucleic acid coding for the Cas protein.

13. The composition according to claim 12, which further comprises guide RNA or nucleic acid coding for the guide RNA.
